# EUROPÄISCHE PATENTSCHRIFT

(11) **EP 0 979 223 B1**
(45) Veröffentlichungstag und Bekanntmachung des Hinweises auf die Patenterteilung: **29.05.2002**
(21) Anmeldenummer: 98922690.7
(22) Anmeldetag: 11.04.1998
(51) Int. Cl.: C07C 51/215, C07C 51/25, C07C 53/08

(54) **VERFAHREN ZUR HERSTELLUNG VON ESSIGSÄURE IN EINER REAKTORKASKADE**
METHOD FOR PRODUCING ACETIC ACID IN A REACTOR CASCADE
PROCEDE DE FABRICATION D'ACIDE ACETIQUE DANS UNE CASCADE DE REACTEURS

(30) Priorität: 23.04.1997 DE 19717075
(43) Veröffentlichungstag der Anmeldung: 16.02.2000
(73) Patentinhaber: Celanese Chemicals Europe GmbH, 60439 Frankfurt am Main (DE)
(72) Erfinder: BORCHERT, Holger, D-67278 Bockenheim (DE); DINGERDISSEN, Uwe, D-64342 Seeheim-Jugenheim (DE); ROESKY, Rainer, D-65929 Frankfurt am Main (DE)
(86) Internationale Anmeldenummer: EP9802126
(87) Internationale Veröffentlichungsnummer: WO9847851

(56) Entgegenhaltungen:
- US-A- 5 300 684

## Beschreibung

Die vorliegende Erfindung betrifft ein verbessertes Verfahren zur Herstellung von Essigsäure durch Oxidation von Ethan, indem die Reaktion in einer Reaktorkaskade durchgeführt wird.

Die katalytische Gasphasenoxidation von Ethan zu Ethylen und Essigsäure ist bekannt. 1978 veröffentlichte die Union Carbide Corporation einen Bericht im Journal of Catalysis 52, 116-132, worin Katalysatoren für die oxidative Dehydrierung von Ethan zu Ethylen beschrieben werden. Mehrere US-Patente (4 250 346, 4 524 236, 4 568 790, 4 899 003 und 4 598 787) wurden für Verfahren erteilt, die die oxidative Dehydrierung von Ethan zu Ethylen bei niedrigen Temperaturen betreffen. Eventuell kommerzialisierbare Verfahren zur oxidativen Dehydrierung von Ethan zu Ethylen werden in The Arabian Journal for Science and Engineering 1985, l10, 353-360, US-4 899 003 und EP-A-0 261 264 offenbart. Essigsäure wird in diesen Verfahren nur als Nebenprodukt gebildet.

Ein Verfahren für die Umsetzung von Ethan zu Essigsäure wird wesentlich durch die Reaktionsbedingungen bestimmt, die für eine maximale Ausbeute an Essigsäure bezogen auf den Eingangsstoff Ethan notwendig sind. Wie in EP-A-0 407 091 offenbart, wird die Umsetzung in der Gasphase bei Temperaturen zwischen 200 und 500°C sowie unter einem erhöhten Druck von bis zu 30 bar durchgeführt. Zur Vermeidung explosionsfähiger Gasmischungen wird Ethan im Überschuß bezogen auf die Sauerstoffmenge in den Reaktor eingespeist. Somit ist der Ethanumsatz und die Essigsäuremenge, die pro Reaktordurchgang erreicht werden können, durch die Sauerstoffkonzentration im Reaktoreingangsgas begrenzt. Weiterhin wird Wasserdampf zum Reaktoreingangsgas eingespeist, was den Vorteil hat, daß die Bildung von Essigsäure zu Lasten der Ethylenbildung begünstigt wird. Der Nachteil ist, daß dadurch eine verdünnte, wäßrige Essigsäure erhalten wird, was erhebliche Kosten für die Aufarbeitung zur konzentrierten Essigsäure zur Folge hat.

Ein Verfahren zur Oxidation von Ethan zu Essigsäure wird in US-5 300 684 offenbart. Dieses Verfahren umfaßt die Einspeisung von Ethan und einem Rückführgas in eine Wirbelbett-Reaktionszone, enthaltend gewirbelten teilchenförmigen festen Oxidationskatalysator, sowie die Einspeisung eines molekularen Sauerstoff enthaltenden Gases, getrennt von der Ethanzuführung. Als gasförmige Nebenprodukte werden bei dem Verfahren Ethylen, Kohlendioxid und Kohlenmonoxid gebildet. Daher schließt das Verfahren weiterhin die Stufen
1) Abkühlen des abgezogenen gasförmigen Produktes aus der Reaktionszone,
2) Abtrennen des Hauptanteils der Essigsäure in flüssiger Form aus den ausströmenden Gasen, und
3) Ausschleusung eines Teilstroms aus dem Rückführgas ein.

Als Vorteile des Wirbelschichtreaktors werden genannt:
1. Auf Grund der Hydrodynamik des Wirbelschichtreaktors wird die Rückvermischung des Gases im Reaktor begünstigt und somit die Selektivität zu Essigsäure zu Lasten der Ethylenbildung begünstigt.
2. Die Vermischung der reaktiven Gase Ethan und Sauerstoff mit dem inerten Reaktionsprodukt Kohlendioxid erlaubt es im Wirbelschichtreaktor, mit höheren Sauerstoffkonzentrationen im Eingangsgas zu arbeiten als in einem Festbettreaktor. Hierdurch wird ein höherer Ethanumsatz pro Reaktordurchgang erzielt.

Der Wirbelschichtreaktor hat aber nach Fluidization Engineering, Butterworth Heinemann, Boston, 1991, S. 10 folgende Nachteile:
1. Die Rückvermischung des Reaktionsgases im Wirbelschichtreaktor führt dazu, daß durch die Weiteroxidation der erwünschten Produkte (in diesem Fall Essigsäure) zu Kohlendioxid und Kohlenmonoxid die Ausbeute verringert wird.
2. Wegen des starken mechanischen Katalysatorabriebs entstehen hohe Kosten für den Katalysator.

Die in US-5 300 684 offenbarte Ausschleusung des Teilstroms aus dem Rückführgas soll eine Anreicherung von Kohlendioxid im Reaktorgas verhindern.

Als Vorteil der Ausschleusung wird genannt, daß hierdurch die energieaufwendige kryogene Abtrennung von Kohlenmonoxid und Ethylen vermieden wird. Sie hat jedoch den Nachteil, daß nicht nur Kohlendioxid, Kohlenmonoxid und Ethylen ausgeschleust werden, sondern auch ein Teil des nicht umgesetzten Ethans. Der Verlust des wertvollen Rohstoffs Ethan führt zu erheblich höheren Materialkosten bezogen auf die Menge der gebildeten Essigsäure.

Ein weiterer Nachteil des Verfahrens ist es, daß die gebildete wäßrige Essigsäure in stark verdünnter Form anfällt. Aus dem in US-5300 684 offenbarten Beispiel ist zu entnehmen, daß die Essigsäurekonzentration lediglich 26 Gewichtsprozent beträgt, so daß erhebliche Kosten für die Aufarbeitung zu konzentrierter Essigsäure entstehen.

Es bestand daher die Aufgabe, ein Verfahren zur Verfügung zu stellen, das es erlaubt, Essigsäure wirtschaftlich durch katalytische Oxidation von Ethan zu erhalten.

Überraschenderweise wurde gefunden, daß die Nachteile der Verfahren nach dem Stand der Technik unterbleiben, wenn die Oxidation von Ethan zu Essigsäure in einer Reaktorkaskade durchgeführt wird.

Gegenstand der Erfindung ist ein Verfahren zur Herstellung von Essigsäure aus Ethan und Sauerstoff oder Sauerstoff enthaltenden Gasen an einem Festbettkatalysator, dadurch gekennzeichnet, daß man
a) Ethan, Sauerstoff oder ein Sauerstoff enthaltendes Gas und ein Rückführgas in einen Reaktor einspeist, der einen Festbettkatalysator enthält,
b) das in Schritt a) erhaltene Reaktorabgas mit Sauerstoff oder einem Sauerstoff enthaltenden Gas mischt, ohne zuvor Wasser oder Essigsäure abzutrennen,
c) das in Schritt b) erhaltene Gasgemisch in einen weiteren Reaktor einspeist der einen Festbettkatalysator enthält,
d) das in Schritt c) erhaltene Reaktorabgas abkühlt,
e) aus dem Schritt d) erhaltenen Gasstrom das darin enthaltene Kohlendioxid ganz oder teilweise entfernt, und
f) den in Schritt e) erhaltenen Gasstrom als Rückführgas nach Schritt a) verwendet.

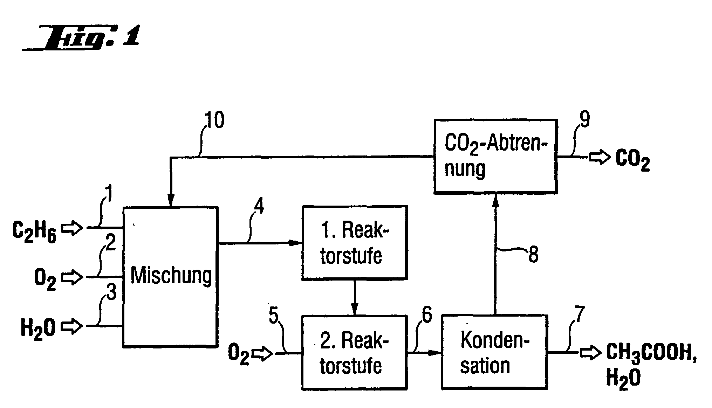

Zur Veranschaulichung ist ein Verfahrensfließbild in Abbildung 1 angegeben, in dem als Beispiel eine zweistufige Reaktion dargestellt ist. Im folgenden werden die einzelnen Verfahrensschritte des erfindungsgemäßen Verfahrens detailliert beschrieben.

Man erhält das Reaktoreingangsgas, indem ein Rückführgas, das überwiegend aus nicht umgesetztem Ethan und Kohlendioxid besteht, mit frischem Ethan und Sauerstoff oder einem molekularen Sauerstoff enthaltenden Gas gemischt und in einen Festbettreaktor eingespeist wird. Das Sauerstoff enthaltende Gas kann Luft oder ein an Sauerstoff reicheres oder ärmeres Gas als Luft sein. Vorteilhaft ist die Verwendung von reinem Sauerstoff, da hierbei die kryogene Abtrennung von Stickstoff aus dem Reaktionsgas entfällt. Weiterhin kann das Eingangsgas Wasserdampf in Konzentrationen von 1 bis 50 Vol% beinhalten. Bevorzugt ist eine Wasserdampfkonzentration von 5 bis 30 Vol%. Der Zusatz von Wasserdampf ermöglicht eine höhere Selektivität bei der Bildung von Essigsäure. Das molare Verhältnis von gesamtem Ethan zu Sauerstoff, das der Reaktionszone zugeführt wird, liegt bevorzugt im Bereich zwischen 2:1 und 10:1, insbesondere zwischen 3:1 und 8:1.

Das Eingangsgas wird durch einen Reaktor geleitet, in dem der Katalysator als Festbettschüttung angeordnet ist. Durch katalytische Oxidation wird Ethan zu Essigsäure oxidiert, wobei durch die geeignete Wahl des Katalysators und der Reaktionsbedingungen als Nebenprodukt überwiegend nur Kohlendioxid gebildet wird und die Bildung anderer gasförmiger Produkte wie Ethylen und Kohlenmonoxid unterbleibt. Die Reaktion wird bei Temperaturen zwischen 150 und 500°C, bevorzugt 200 bis 400°C durchgeführt. Der Druck kann atmosphärisch oder superatmosphärisch sein und bevorzugt im Bereich zwischen 1 und 50 bar, insbesondere 2 bis 30 bar liegen. Die Verweilzeit im Reaktor wird so eingestellt, daß ein weitergehender Sauerstoffumsatz erreicht wird. Als Katalysator verwendet man vorzugsweise Katalysatoren, wie sie in den deutschen Patentanmeldungen 19630832.1 oder 19620542.5 beschrieben sind, worauf hiermit Bezug genommen wird.

DE-19620542.5 offenbart einen Katalysator, der die Elemente Mo, Pd, Re, X und Y in den Grammatomverhältnissen a:b:c:d:e in Kombination mit Sauertstoff enthält

MoₐPd_{b}Re_{c}X_{d}Yₑ

und die Symbole X, Y folgende Bedeutung haben:
X = Cr, Mn, Nb, B, Ta, Ti, V und/oder W
Y = Bi, Ce, Co, Te, Fe, Li, K, Na, Rb, Be, Mg, Ca, Sr, Ba, Ni, P, Pb, Sb, Si, Sn, Tl und/oder U. Die Indizes a, b, c, d und e stehen für die Grammatomverhältnisse der entsprechenden Elemente, wobei
a=1, b>0, c>0, d=0,05 bis 2 und e=0 bis 3 ist.

DE-19630832.1 offenbart einen Katalysator, der die Elemente Mo, Pd, X und Y in den Grammatomverhältnissen a:b:c:d in Kombination mit Sauerstoff enthält

MoₐPd_{b}X_{c}Y_{d}

und die Symbole X, Y folgende Bedeutung haben:
X = Cr, Mn, Nb, Ta, Ti, V, Te und/oder W
Y = B, Al, Ga, In, Pt, Zn, Cd, Bi, Ce, Co, Cu, Rh, Ir, Au, Ag, Fe, Ru, Os, K, Rb, Cs, Mg, Ca, Sr, Ba, Zr, Hf, Ni, P, Pb, Sb, Si, Sn, TI, und/oder U. Die Indizes a, b, c und d stehen für die Grammatomverhältnisse der entsprechenden Elemente, wobei
a=1, b>0, c>0, d=0 bis 2 ist.

Es ist auch möglich, einen wolframhaltigen Katalysator, der die Elemente W, X, Y und Z in den Grammatomverhältnissen a:b:c:d in Kombination mit Sauerstoff erhält

WₐX_{b}Y_{c}Z_{d}

für das erfindungsgemäße Verfahren zu verwenden, worin
- X: eines oder mehrere Elemente ausgewählt aus der Gruppe Pd, Pt, Ag und/oder Au,
- Y: eines oder mehrere Elemente ausgewählt aus der Gruppe V, Nb, Cr, Mn, Fe, Sn, Sb, Cu, Zn, U, Ni und/oder Bi,
- Z: eines oder mehrere Elemente ausgewählt aus der Gruppe Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As und/oder Te,
- a: 1,
- b: eine Zahl größer als 0,
- c: eine Zahl größer als 0, und
- d: eine Zahl von 0 bis 2 ist.

Bei der Durchführung des erfindungsgemäßen Verfahrens liegt die Selektivität der Oxidation von Ethan zu Essigsäure bei ≥ 60 Mol%, vorzugsweise ≥ 75 Mol%, insbesondere ≥ 80 Mol%, bei einem Ethanumsatz pro Reaktorstufe von > 4%, vorzugsweise > 5%, insbesondere > 6%. Ein weiterer Vorteil des Verfahrens ist, daß als Nebenprodukt nahezu ausschließlich Kohlendioxid gebildet wird, während die Bildung von Ethylen oder Kohlenmonoxid vernachlässigbar ist. Die Summe der Selektivitäten für die Entstehung von Ethylen und Kohlenmonoxid liegt bei ≤ 5 Mol%, vorzugsweise ≤ 4 Mol%, insbesodnere ≤ 3 Mol%.

Das die erste Reaktorstufe verlassende Reaktorabgas wird mit Sauerstoff oder einem Sauerstoff enthaltenden Gas gemischt, und, ohne zuvor Wasser und Essigsäure auszukondensieren, in einen zweiten Reaktor eingeleitet. Die Verwendung mehrerer hintereinander angeordneter Reaktorstufen mit Einspeisung von Sauerstoff enthaltendem Gas zwischen den Reaktorstufen ohne Abtrennung der Essigsäure ermöglicht einen hohen Ethanumsatz und verringert die im Kreis geführte Gasmenge. Man erhält im Vergleich zu einem einstufigen Reaktor nach der letzten Reaktorstufe eine wäßrige Essigsäure, die eine höhere Konzentration besitzt. Hierdurch wird die Aufarbeitung zur konzentrierten Essigsäure wesentlich vereinfacht. Die Anzahl der Reaktorstufen richtet sich nach den in den einzelnen Stufen erreichten Ethan- und Sauerstoffumsätzen. Vorzugsweise beträgt sie ≥ 2 Stufen. Mit steigender Anzahl der Reaktorstufen werden eine Erhöhung der Ethanumsätze und eine Verringerung der im Kreis geführten Gasmenge erreicht. Zudem steigt die Konzentration der wäßrigen Essigsäure nach der letzten Reaktorstufe. Andererseits steigen jedoch die Kosten für die Reaktoren. Somit ist eine Optimierung unter wirtschaftlichen Gesichtspunkten hinsichtlich der Anzahl der Reaktorstufen in Abhängigkeit der in den Reaktorstufen erzielten Ethan- und Sauerstoffumsätze notwendig.

Bei Verwendung der oben genannten Katalysatoren wird die in der ersten Reaktorstufe gebildete Essigsäure in den darauf folgenden Reaktorstufen nicht weiteroxidiert. Es ist deshalb nicht notwendig, die Essigsäure nach den einzelnen Stufen aus dem Gasstrom durch Kondensation zu entfernen.

Nach der letzten Reaktorstufe wird das Reaktorabgas abgekühlt, wobei Wasser und die gebildete Essigsäure auskondensieren. Der nach der Kondensation erhaltene Gasstrom besteht überwiegend aus Ethan und Kohlendioxid neben sehr geringen Mengen Ethylen und Kohlenmonoxid.

Durch Wäscher oder Membranen wird das Kohlendioxid, das während der Reaktion gebildet wird, aus dem Gasstrom entfernt, um seine Akkumulation im Kreisgas zu vermeiden. Vorzugsweise wird Kohlendioxid durch eine Wäsche entfernt. Im Kreisgas verbleiben dann nur Ethan und der restliche Anteil des Kohlendioxids neben geringen Mengen an Ethylen und Kohlenmonoxid.

Durch die bei der Durchführung des erfindungsgemäßen Verfahrens vemachlässigbare Bildung von Ethylen und Kohlenmonoxid entfällt die energieaufwendige kryogene Abtrennung dieser Gase vom nicht umgesetzten Ethan. Ebenso entfällt die in US-5 300 684 beschriebene Ausschleusung eines Teilstroms aus dem Rückführgas. Hierdurch wird ein Verlust von wertvollem Ethan verhindert.

Nach Abtrennung des in der Reaktion gebildeten Kohlendioxids wird das Kreisgas an den ersten Reaktoreingang zurückgeführt, mit frischem Ethan, Sauerstoff sowie Wasserdampf vermischt und wieder in die erste Reaktorstufe eingespeist.

### Beispiel

Ein spezifisches Beispiel ist in Tabelle 1 zusammengestellt, wobei die Kennzeichnungen der Mengenströme die gleichen sind wie in Abbildung 1. In diesem Beispiel beträgt die Temperatur in den Reaktorstufen etwa 280°C und der Druck etwa 15 bar. Verwendet wurde ein Katalysator, der die Elemente Mo, Pd, V, Nb, Sb und Ca (MOₐPd_{b}V_{c}Nb_{d}SbₑCa_{f}) in den Grammatomverhältnissen a:b:c:d:e:f = 1 : 0,0005 : 0,36 : 0,03: 0,01 : 0,01 in Kombination mit Sauerstoff enthält. Es ist festzuhalten, daß die Ausbeute an Essigsäure bezogen auf frisch eingespeistes Ethan sehr hoch ist, da keine Ausschleusung von Ethan enthaltenden Gas aus dem Rückführstrom durchgeführt wird. Zudem ist die Konzentration der erzeugten wäßrigen Essigsäure mit 45 Gewichts-% sehr hoch.

**Tabelle 1:**

| Mengenströme zum Verfahrensfließbild (Abbildung 1) | | | | | | | | | | |
|---|---|---|---|---|---|---|---|---|---|---|
| Substanz | Mengenströme in 1000 kg/Stunde | | | | | | | | | |
| | 1 | 2 | 3 | 4 | 5 | 6 | 7 | 8 | 9 | 10 |
| C₂H₆ | 19,6 | 0 | 0 | 77,3 | 0 | 60,0 | 0,2 | 59,9 | 0 | 68,0 |
| O₂ | 0 | 15,3 | 0 | 33,0 | 15,3 | 2,4 | 0 | 2,4 | 0 | 2,4 |
| H₂O | 0 | 0 | 26,3 | 26,3 | 0 | 38,5 | 37,9 | 0,6 | 0,6 | 0 |
| CH₃COOH | 0 | 0 | 0 | 0 | 0 | 31,3 | 30,7 | 0,5 | 0,5 | 0 |
| CO₂ | 0 | 0 | 0 | 103,0 | 0 | 107,7 | 0,3 | 107,3 | 4,6 | 103,0 |

## Patentansprüche

1. Verfahren zur Herstellung von Essigsäure aus Ethan und Sauerstoff oder Sauerstoff enthaltenden Gasen an einem Festbettkatalysator, **dadurch gekennzeichnet, daß** man
a) Ethan, Sauerstoff oder ein Sauerstoff enthaltendes Gas und ein Rückführgas in einen Reaktor einspeist, der einen Festbettkatalysator enthält,
b) das in Schritt a) erhaltene Reaktorabgas mit Sauerstoff oder einem Sauerstoff enthaltenden Gas mischt, ohne zuvor Wasser oder Essigsäure abzutrennen,
c) das in Schritt b) erhaltene Gasgemisch in einen weiteren Reaktor einspeist der einen Festbettkatalysator enthält,
d) das in Schritt c) erhaltene Reaktorabgas abkühlt,
e) aus dem Schritt d) erhaltenen Gasstrom das darin enthaltene Kohlendioxid ganz oder teilweise entfernt,
f) den in Schritt e) erhaltenen Gasstrom als Rückführgas nach Schritt a) verwendet.

2. Verfahren nach Anspruch 1, **dadurch gekennzeichnet, daß** man das in Schritt c) erhaltene Reaktorabgas vor der Abkühlung in Schritt d) in einen oder mehrere weitere Reaktoren einspeist, und vor jeder Einspeisung das Reaktorabgas mit Sauerstoff oder Sauerstoff enthaltenden Gasen mischt.

3. Verfahren nach Anspruch 1 und/oder 2, **dadurch gekennzeichnet, daß** das Reaktoreingangsgas 1 bis 50 Vol.-%, insbesondere 5 bis 30 Vol.-% Wasserdampf enthält.

4. Verfahren nach einem oder mehreren der Ansprüche 1 bis 3, **dadurch gekennzeichnet, daß** das molare Verhältnis von Ethan zu Sauerstoff im Reaktoreingangsgas zwischen 2:1 und 10:1, insbesondere zwischen 3:1 und 8:1 liegt.

5. Verfahren nach einem oder mehreren der Ansprüche 1 bis 4, **dadurch gekennzeichnet, daß** die Reaktion bei Temperaturen zwischen 150 und 500°C, insbesondere zwischen 200 und 400°C durchgeführt wird.

6. Verfahren nach einem oder mehreren der Ansprüche 1 bis 5, **dadurch gekennzeichnet, daß** die Reaktion bei Drucken zwischen 1 und 50 bar, insbesondere zwischen 2 und 30 bar durchgeführt wird.

7. Verfahren nach einem oder mehreren der Ansprüche 1 bis 6, **dadurch gekennzeichnet, daß** ein Katalysator, der die Elemente Mo, Pd, Re, X und Y in den Grammatomverhältnissen a:b:c:d:e in Kombination mit Sauerstoff enthält
MoₐPd_{b}Re_{c}X_{d}Yₑ
wobei die Symbole X und Y
X = Cr, Mn, Nb, B, Ta, Ti, V und/oder W,
Y = Bi, Ce, Co, Te, Fe, Li, K, Na, Rb, Be, Mg, Ca, Sr, Ba, Ni, P, Pb, Sb, Si, Sn, Tl und/oder U bedeuten,
und die Indizes a, b, c, d und e für die Grammatomverhältnisse der entsprechenden Elemente stehen, wobei a=1, b>0, c>0, d=0,05 bis 2 und e=0 bis 3 ist, verwendet wird.

8. Verfahren nach einem oder mehreren der Ansprüche 1 bis 6, **dadurch gekennzeichnet, daß** ein Katalysator, der die Elemente Mo, Pd, X und Y in den Grammatomverhältnissen a:b:c:d in Kombination mit Sauerstoff enthält
MoₐPd_{b}X_{c}Y_{d}
wobei die Symbole X und Y X = Cr, Mn, Nb, Ta, Ti, V, Te und/oder W, Y = B, Al, Ga, In, Pt, Zn, Cd, Bi, Ce, Co, Cu, Rh, Ir, Au, Ag, Fe, Ru, Os, K, Rb, Cs, Mg, Ca, Sr, Ba, Zr, Hf, Ni, P, Pb, Sb, Si, Sn, Tl, und/oder U bedeuten, und die. Indizes a, b, c und d für die Grammatomverhältnisse der entsprechenden Elemente stehen, wobei a=1, b>0, c>0 und d=0 bis 2 ist, verwendet wird.

9. Verfahren nach einem oder mehreren der Ansprüche 1 bis 6, **dadurch gekennzeichnet, daß** ein wolframhaltiger Katalysator, der die Elemente W, X, Y und Z in den Grammatomverhältnissen a:b:c:d in Kombination mit Sauerstoff enthält
WₐX_{b}Y_{c}Z_{d}(I)
worin
X eines oder mehrere Elemente ausgewählt aus der Gruppe Pd, Pt, Ag und/oder Au,
Y eines oder mehrere Elemente ausgewählt aus der Gruppe V, Nb, Cr, Mn, Fe, Sn, Sb, Cu, Zn, U, Ni und/oder Bi,
Z eines oder mehrere Elemente ausgewählt aus der Gruppe Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As und/oder Te,
a 1,
b eine Zahl größer als 0,
c eine Zahl größer als 0, und
d eine Zahl von 0 bis 2 ist
bedeuten, verwendet wird.

## Claims

1. A process for the preparation of acetic acid from ethane and oxygen or oxygen-containing gases on a fixed bed catalyst, which comprises
a) feeding ethane, oxygen or an oxygen-containing gas and a recycled gas into a reactor which contains a fixed bed catalyst,
b) mixing the reactor exit gas obtained in step a) with oxygen or an oxygen-containing gas without removing water or acetic acid beforehand,
c) feeding the gas mixture obtained in step b) into another reactor which contains a fixed bed catalyst,
d) cooling the reactor exit gas obtained in step c),
e) removing all or part of the carbon dioxide present in the gas stream obtained in step d), and
f) using the gas stream obtained in step e) as recycled gas for step a).

2. The process as claimed in claim 1, wherein the reactor exit gas obtained in step c) is, before the cooling in step d), fed into one or more other reactors and, before each feeding in, the reactor exit gas is mixed with oxygen or oxygen-containing gases.

3. The process as claimed in claim 1 and/or 2, wherein the gas entering the reactor comprises 1 to 50% by volume, in particular 5 to 30% by volume, of steam.

4. The process as claimed in one or more of claims 1 to 3, wherein the molar ratio of ethane to oxygen in the gas entering the reactor is between 2:1 and 10:1, in particular between 3:1 and 8:1.

5. The process as claimed in one or more of claims 1 to 4, wherein the reaction is carried out at temperatures between 150 and 500°C, in particular between 200 and 400°C.

6. The process as claimed in one or more of claims 1 to 5, wherein the reaction is carried out under pressures between 1 and 50 bar, in particular between 2 and 30 bar.

7. The process as claimed in one or more of claims 1 to 6, wherein a catalyst which comprises the elements Mo, Pd, Re, X and Y in the gram-atom ratios a:b:c:d:e in combination with oxygen
MoₐPd_{b}Re_{c}X_{d}Yₑ
where the symbols X and Y mean
X = Cr, Mn, Nb, B, Ta, Ti, V and/or W,
Y = Bi, Ce, Co, Te, Fe, Li, K, Na, Rb, Be, Mg, Ca, Sr, Ba, Ni, P, Pb, Sb, Si, Sn, Tl and/or U,
and the indices a, b, c, d and e are the gram-atom ratios of the appropriate elements, where a = 1, b > 0, c > 0, d = 0.05 to 2 and e = 0 to 3, is used.

8. The process as claimed in one or more of claims 1 to 6, wherein a catalyst which comprises the elements Mo, Pd, X and Y in the gram-atom ratios a:b:c:d in combination with oxygen
MoₐPd_{b}X_{c}Y_{d}
where the symbols X and Y mean
X = Cr, Mn, Nb, Ta, Ti, V, Te and/or W, Y = B, Al, Ga, In, Pt, Zn, Cd, Bi, Ce, Co, Cu, Rh, Ir, Au, Ag, Fe, Ru, Os, K, Rb, Cs, Mg, Ca, Sr, Ba, Zr, Hf, Ni, P, Pb, Sb, Si, Sn, TI, and/or U, and the indices a, b, c and d are the gram-atom ratios of the appropriate elements, where a = 1, b > 0, c > 0 and d = 0 to 2, is used.

9. The process as claimed in one or more of claims 1 to 6, wherein a tungsten-containing catalyst which comprises the elements W, X, Y and Z in the gram-atom rations a:b:c:d in combination with oxygen
WₐX_{b}Y_{c}Z_{d}(I)
in which
X is one or more elements selected from the group of Pd, Pt, Ag and/or Au,
Y is one or more elements selected from the group of V, Nb, Cr, Mn, Fe, Sn, Sb, Cu, Zn , U, Ni and/or Bi,
Z is one/or more elements selected from the group of Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As and/or Te,
a is 1,
b is a number greater than 0,
c is a number greater than 0, and
d is a number from 0 to 2, is used.

## Revendications

1. Procédé pour la préparation d'acide acétique à partir d'éthane et d'oxygène ou de gaz contenant de l'oxygène sur un catalyseur en lit fixe, **caractérisé en ce que**
a) on introduit de l'éthane, de l'oxygène ou un gaz contenant de l'oxygène et un gaz recyclé dans un réacteur, qui contient un catalyseur en lit fixe,
b) on mélange le gaz de sortie du réacteur obtenu dans l'étape a) avec de l'oxygène ou un gaz contenant de l'oxygène sans séparer préalablement l'eau ou l'acide acétique,
c) on introduit le mélange gazeux obtenu dans l'étape b) dans un autre réacteur qui contient un catalyseur en lit fixe,
d) on refroidit le gaz de sortie du réacteur obtenu dans l'étape c),
e) on élimine totalement ou partiellement du courant gazeux obtenu dans l'étape d) le dioxyde de carbone qui y est contenu,
f) on utilise le courant gazeux obtenu dans l'étape e) comme gaz recyclé pour l'étape a).

2. Procédé selon la revendication 1, **caractérisé en ce que** l'on introduit le gaz de sortie du réacteur obtenu dans l'étape c) avant le refroidissement dans l'étape d) dans un ou plusieurs autres réacteurs et on mélange le gaz de sortie du réacteur avec de l'oxygène ou des gaz contenant de l'oxygène avant chaque introduction.

3. Procédé selon la revendication 1 et/ou 2, **caractérisé en ce que** le gaz d'entrée du réacteur contient de 1 à 50 % en volume, en particulier de 5 à 30 % en volume de vapeur d'eau.

4. Procédé selon l'une ou plusieurs quelconques des revendications 1 à 3, **caractérisé en ce que** le rapport molaire de l'éthane à l'oxygène dans le gaz d'entrée du réacteur est compris entre 2:1 et 10:1, en particulier entre 3:1 et 8:1.

5. Procédé selon l'une ou plusieurs quelconques des revendications 1 à 4, **caractérisé en ce que** la réaction est réalisée à des températures comprises entre 150 et 500°C, en particulier entre 200 et 400°C.

6. Procédé selon l'une ou plusieurs quelconques des revendications 1 à 5, **caractérisé en ce que** la réaction est réalisée à des pressions comprises entre 1 et 50 bar, en particulier entre 2 et 30 bar.

7. Procédé selon l'une ou plusieurs quelconques des revendications 1 à 6, **caractérisé en ce que** l'on utilise un catalyseur qui contient les éléments Mo, Pd, Re, X et Y dans les rapports d'atomes grammes a:b:c:d:e en combinaison avec de l'oxygène
MoₐPd_{b}Re_{c}X_{d}Yₑ
les symboles X et Y représentant
X = Cr, Mn, Nb, B, Ta, Ti, V et/ou W,
Y = Bi, Ce, Co, Te, Fe, Li, K, Na, Rb, Be, Mg, Ca, Sr, Ba, Ni, P, Pb, Sb, Si, Sn, Tl et/ou U,
et les indices a, b, c, d et e représentant les rapports d'atomes grammes des éléments correspondants, a=1, b>0, c>0, d=0,05 à 2 et e=0 à 3.

8. Procédé selon l'une ou plusieurs quelconques des revendications 1 à 6, **caractérisé en ce que** l'on utilise un catalyseur qui contient les éléments Mo, Pd, X et Y dans les rapports d'atomes grammes a:b:c:d en combinaison avec de l'oxygène
MoₐPd_{b}X_{c}Y_{d}
dans laquelle les symboles X et Y représentant
X = Cr, Mn, Nb, Ta, Ti, V, Te et/ou W,
Y = B, Al, Ga, In, Pt, Zn, Cd, Bi, Ce, Co, Cu, Rh, Ir, Au, Ag, Fe, Ru, Os, K, Rb, Cs, Mg, Ca, Sr, Ba, Zr, Hf, Ni, P, Pb, Sb, Si, Sn, Tl, et/ou U, et
les indices a, b, c et d représentant les rapports d'atomes grammes des éléments correspondants, a=1, b>0, c>0 et d=0 à 2.

9. Procédé selon l'une ou plusieurs quelconques des revendications 1 à 6, **caractérisé en ce que** l'on utilise un catalyseur contenant du tungstène, lequel contient les éléments W, X, Y et Z dans les rapports d'atomes grammes a:b:c:d en combinaison avec de l'oxygène
WₐX_{b}Y_{c}Z_{d}(I)
où
X représente un ou plusieurs éléments choisis parmi Pd, Pt, Ag et/ou Au,
Y représente un ou plusieurs éléments choisis parmi V, Nb, Cr, Mn, Fe, Sn, Sb, Cu, Zn, U, Ni et/ou Bi,
Z représente un ou plusieurs éléments choisis parmi Li, Na, K, Rb, Cs, Be, Mg, Ca, Sr, Ba, Sc, Y, La, Ti, Zr, Hf, Ru, Os, Co, Rh, Ir, B, AI, Ga, In, TI, Si, Ge, Pb, P, As et/ou Te,
a=1,
b est un nombre supérieur à 0,
c est un nombre supérieur à 0, et
d est un nombre compris entre 0 et 2.
